# EUROPEAN PATENT SPECIFICATION

(11) **EP 4 491 095 B1**
(45) Date of publication and mention of the grant of the patent: **25.03.2026**
(21) Application number: 24000083.6
(22) Date of filing: 27.06.2024
(51) Int. Cl.: A61B 1/12, A61L 2/18, A61B 90/70

(54) **METHOD AND SYSTEM FOR CONTROLLING THE TEMPERATURE IN AN ENDOSCOPE REPROCESSING MACHINE**
VERFAHREN UND SYSTEM ZUR TEMPERATURREGELUNG IN EINER WASCHMASCHINE FÜR ENDOSKOPE
PROCEDURE ET SYSTEME DE CONTROLE DE LA TEMPERATURE DANS UNE MACHINE A LAVER LES ENDOSCOPES

(30) Priority: 12.07.2023 IT 202300014655
(43) Date of publication of application: 15.01.2025
(73) Proprietor: Nuova S.B. System S.r.l., 20092 Cinisello Balsamo (MI) (IT)
(72) Inventor: Bongiovanni, Davide, 20092 Cinisello Balsamo (MI) (IT)
(74) Representative: Aprà, Mario

(56) References cited:
- DE-A1- 3 712 701
- DE-A1- 4 003 987
- US-A1- 2012 227 767

## Description

The present invention refers to a method for controlling the temperature of the water used for cleansing an endoscope to be reprocessed in an endoscope washer machine, comprising a tank made of medical grade polymer material. The invention also refers to a system for implementation of the aforesaid method.

It is specified that the invention can be specifically applied in an endoscope washer machine made of medical grade polymer material, in particular in a machine of the type specified comprising a treatment tank made of medical grade polymer material.

### Background

The standard UNI EN ISO 15883 establishes the process steps for reprocessing endoscopes, listing the crucial steps to be followed to obtain complete removal of organic material from the instrument and perfect cleanliness and disinfection.

These steps include cleansing, which is carried out using an enzymatic detergent.

The temperature of the water used for washing with the detergent is considered suitable if within the range from 30 to 40°C. In this temperature range, the enzymes optimize and accelerate their catalytic reaction, maximizing removal of the organic material present on the surface and within the channels of endoscopes which are reprocessed after use. At the current state of the art, an endoscope washer machine made of polymeric material can use water at the correct temperature, connecting the hot water intake of the machine with the specific connection provided in the water supply network of the healthcare structure in which the machine is installed.

In case of the use of water with a temperature exceeding 40°C in the pipes of the water supply network, the endoscope washer machine of the type specified can use an suitable thermostatic mixer to obtain water at the correct temperature; however, in this case, it is necessary to hydraulically connect the hot water intake of the machine both to the hot water network and to the cold water network of the healthcare structure.

Other types of endoscope washer machines allow the water to be heated inside the machine, through systems that do not store water, which allow it to be heated to the correct temperature.

The document DE 40 03 987 A1 discloses a system for controlling the temperature of the water used to cleanse an endoscope to be reprocessed in an endoscope washer machine of the type specified above.

### Technical problem underlying the invention

At the current state of the art there are no known endoscope washer machines made of polymeric material, having an internal system for heating the water for cleansing endoscopes integrated with the machine by means of a storage tank. A part of currently known machines can only be installed through connection both to the hot water and to the cold water of the water supply network of the healthcare structure, while the remaining part of the machines has an internal resistor that allows water to be heated, by means of a process which, however, is long and somewhat inefficient.

### Summary of the invention

An object of the present invention is to provide a method for controlling the temperature of the water used for cleansing an endoscope to be reprocessed in an endoscope washer machine, comprising a tank made of medical grade polymer material, which allows the water to be heated and used at the optimal temperature during the cleansing step in the endoscope reprocessing process.

Another object of the present invention is to provide a system which allows the aforesaid method to be implemented.

In view of these objects, the present invention provides a method for controlling the temperature of the water used for cleansing an endoscope to be reprocessed in an endoscope washer machine, comprising a tank made of medical grade polymer material, the essential feature of which forms the subject matter of claim 1.

The present invention also provides a system for implementation of the method indicated above, the essential feature of which forms the subject matter of claim 4. Other advantageous features form the subject matter of the dependent claims.

### Description of the drawing

In the drawing:

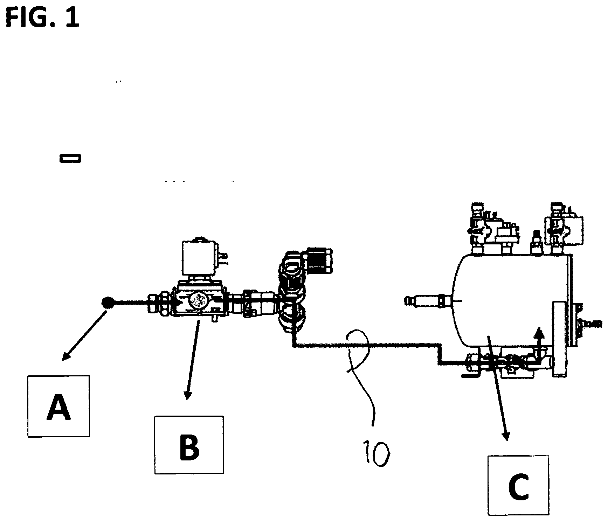

- Fig. 1 is a schematic view, with parts in section, illustrating a part of an endoscope washer machine in which the method according to the invention is implemented by means of the system according to the invention;

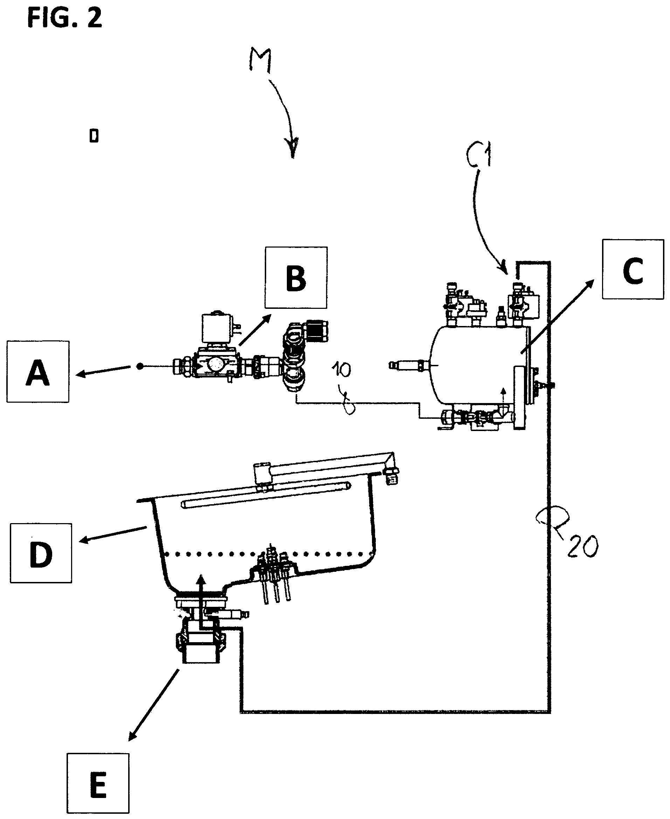

- Fig. 2 is a schematic view, with parts in section, illustrating in its entirety the endoscope washer machine in which the method according to the invention is implemented by means of the system according to the invention.

With reference to the drawing, the method for controlling the temperature of the water used for cleansing an endoscope to be reprocessed is implemented in an endoscope washer machine M, comprising a tank D made of medical grade polymer material, wherein:
- said tank D is provided with an opening E in the bottom of the tank for discharging the water from said tank, and
- said discharge opening E is configured to be selectively opened and allow the outflow of water and closed to prevent the outflow of water from said tank D.

The method according to the invention is characterized in that it comprises the steps of:
- providing said endoscope washer machine M with an internal hot water storage tank C;
- providing said storage tank C with heating means of the water contained in the storage tank;
- providing in said endoscope washer machine M a first hydraulic line 10 of water to be heated, coming from an external water supply network (cold water);
- providing in said first hydraulic line 10 of water to be heated:
   - a water intake A hydraulically connected with respect to said external water supply network,
   - a first solenoid valve B to shut off water flow, hydraulically branched downstream of said water intake A and maintained in a permanent hydraulically open position;
   - a second solenoid valve downstream of said first solenoid valve B and hydraulically connected to a water inlet of said storage tank C to load water in said storage tank C, wherein said second solenoid valve is configured to be selectively switched into a hydraulically open position, in which it allows the water to be heated to flow from said water intake A to said storage tank C, and into a closed position, in which it shuts off the flow of water to be heated from said water intake A to said storage tank C;
- switching said second solenoid valve into said open position, and filling the storage tank C with water from said external water supply network,
- after said storage tank C has been filled, switching said second solenoid valve into said closed position, in which further water cannot enter said storage tank C through said first hydraulic line 10;
- heating the water contained in said storage tank C by means of said heating means up to a temperature of 60°C or higher, maintaining this temperature for at least the time required to eliminate all possible proliferation of bacteria inside said storage tank C;
- switching said second solenoid valve into said open position, in which water from said external water supply network is conveyed inside said storage tank C from said water intake A through said first hydraulic line 10;
- mixing water from said external water supply network entering in the storage tank C through said first hydraulic line 10 with the hot water present in the storage tank C, to take the water mixed in said storage tank C to a temperature ranging from 30°C to 40°C;
- providing a hot water outlet C1 of said storage tank C and hydraulically connecting said hot water outlet C1 of said storage tank C with said tank D by means of a second hydraulic line 20, which passes, in a sealed manner, through said discharge opening E of said tank D and flows into said tank D;
- providing said water outlet C1 with flow shut-off valve means, configured to be switched selectively into a hydraulically open position, in which they allow the hot water to flow from said storage tank C through said second hydraulic line 20, and into a hydraulically closed position, in which they shut off the flow of hot water from said storage tank C through said second hydraulic line 20;
- switching said valve means of said water outlet C1 of said storage tank C into a hydraulically open position and closing said discharge opening E of said tank D;
- pushing the hot water contained in the storage tank C and having a temperature ranging from 30°C to 40°C through said water outlet C1 and said second hydraulic line 20 to said tank D, passing through the discharge opening E of said tank D, by means of a difference in pressure of the cold water entering said storage tank C through said first hydraulic line 10;
- filling the tank D to the desired level with hot water having a temperature ranging from 30°C to 40°C;
- after reaching the desired level of hot water in the tank D, switching said second solenoid valve into the hydraulically closed position and switching said valve means of said water outlet C1 of said storage tank C into the hydraulically closed position;
- utilizing the hot water contained in the tank D to cleanse an endoscope to be reprocessed arranged in said tank D;
- after cleansing of said endoscope to be reprocessed, opening said discharge opening E of said tank D and discharging the water from said tank D.

Moreover, the method according to the invention is characterized by the step of providing a hydraulic branch of said first hydraulic line 10, upstream of said second solenoid valve to said tank D, and providing said hydraulic branch with filter means, with a flow sensor and with a third solenoid valve, so that, when said second solenoid valve is switched into the hydraulically closed position, said hydraulic branch provides a passage of network water (cold water) from said network intake A directly into the wash tank D, to carry out at least one of the following operations: prewash, intermediate rinse, disinfection and final rinse of an endoscope to be reprocessed in said tank D.

Further, the method according to the invention is characterized by the step of configuring said storage tank C as boiler.

Moreover, the present invention provides a system for controlling the temperature of the water used to cleanse an endoscope to be reprocessed in an endoscope washer machine M, comprising a tank D made of medical grade polymer material, wherein:
- said tank D is provided with an opening E in the bottom of the tank for discharging the water from said tank, and
- said discharge opening E is configured to be selectively opened and allow the outflow of water and closed to prevent the outflow of water from said tank D.

The system according to the present invention is characterized in that:
- a hot water storage tank C is arranged inside said endoscope washer machine M;
- heating means of the water contained in said storage tank C are arranged in said storage tank C;
- a first hydraulic line 10 of water to be heated is arranged in said endoscope washer machine M, is branched from an external water supply network (cold water) and comprises:
   - a water intake A hydraulically connected with respect to said external water supply network,
   - a first solenoid valve B to shut off water flow, hydraulically branched downstream of said water intake A and maintained in a permanent hydraulically open position;
   - a second solenoid valve arranged downstream of said first solenoid valve B and hydraulically connected to a water inlet of said storage tank C to load water into said storage tank C, wherein said second solenoid valve is configured to be selectively switched into a hydraulically open position, in which it allows the water to be heated to flow from said water intake A to said storage tank C, and into a hydraulically closed position, in which it shuts off the flow of water to be heated from said water intake A to said storage tank C;
   - said heating means are configured to heat the water contained in said storage tank C to a temperature of 60°C or higher and maintain this temperature at least for the time required to eliminate all possible proliferation of bacteria inside said storage tank C;
   - wherein, in a first operating arrangement:
      - said storage tank C is filled with water from the external water supply network, by means of said first hydraulic line 10 and said second solenoid valve switched into the hydraulically open position,
      - said second solenoid valve is switched into the hydraulically closed position and the water contained in said storage tank C is heated to a temperature of the water of 60°C or higher,
      - the water is maintained at a temperature of 60°C or higher for the time required to eliminate all possible proliferation of bacteria inside said storage tank C,
   - wherein, in a second and subsequent operating arrangement:
      - said second solenoid valve is switched into said hydraulically open position allowing the water of said external water supply network to flow inside said storage tank C from said water intake A through said first hydraulic line 10, mixing with the hot water present in the storage tank C, wherein the temperature of the water mixed ranges from 30°C to 40°C;
   - a hot water outlet C1 is arranged in said storage tank C and is hydraulically connected with said tank D by means of a second hydraulic line 20, which passes, in a sealed manner, through said discharge opening E of said tank D and flows out into said tank D;
   - water flow shut-off valve means are arranged in said water outlet C1 and are configured to be selectively switched into a hydraulically open position, in which they allow hot water to flow through said second hydraulic line 20, and into a hydraulically closed position, in which they shut off the flow of hot water through said second hydraulic line 20;
   - wherein, in a third and subsequent operating arrangement:
      - said valve means of said water outlet C1 of said storage tank C are arranged in a hydraulically open position and said discharge opening E of said tank D is closed, so that the hot water mixed in the storage tank C and maintained at a temperature ranging from 30°C to 40°C is pushed through said water outlet C1 and said second hydraulic line 20 into said tank D, passing through the discharge opening E of the tank D, by means of a difference in pressure of the cold water entering said storage tank C through said first hydraulic line 10, and fills the tank D with hot water at a temperature ranging from 30°C to 40°C to the desired level;
      - after reaching the desired level of hot water at the temperature ranging from 30°C to 40°C in said tank D, said second solenoid valve is switched into the hydraulically closed position and said water outlet C1 of said storage tank C is switched into the hydraulically closed position;
      - the hot water contained in the tank D at a temperature ranging from 30°C to 40°C allows correct cleansing of an endoscope to be reprocessed arranged in the tank D;
      - after cleansing of the endoscope to be reprocessed and opening of said discharge opening E of said tank D, the water utilized for cleansing in said tank D is discharged through said opening E.

Moreover, the system according to the invention is characterized in that a hydraulic branch of said first hydraulic line 10 is arranged upstream of said second solenoid valve and extends into said tank D, wherein said hydraulic branch is provided with filter means, with a flow sensor and with a third solenoid valve, so that, when said second solenoid valve is switched into the hydraulically closed position, said hydraulic branch provides a passage of network water (cold water) from said network intake A directly into said washing tank D, to carry out at least the following operations: prewash, intermediate rinse, disinfection and final rinse of a endoscope to be reprocessed in said tank D.

Further, the system according to the invention is characterized in that said storage tank C is configured as a boiler.

### Advantages of the invention

The method and the relative system according to the invention allow the endoscope washer machine of the type specified to depend solely on the cold water connection of the healthcare structure in which the machine is installed, and allow heating of the cold water to be obtained an a completely efficient and rapid manner.

Moreover, considering the temperature to which the water is preheated inside the machine, the method and relative system according to the invention are completely safe and protected from the possible proliferation of bacteria, which are eliminated by the hot water.

As is apparent from the above, the present invention achieves the objects set forth in the introduction of this description, in a simple and efficient way.

## Claims

1. Method for controlling the temperature of the water used for cleansing an endoscope to be reprocessed in an endoscope washer machine (M) comprising a tank (D) made of medical grade polymer material, wherein:
- said tank (D) is provided with an opening (E) in the bottom of the tank for discharging the water from said tank, and
- said discharge opening (E) is configured to be selectively opened and allow the outflow of water and closed to prevent the outflow of water from said tank (D);
and the method comprises the steps of:
- providing said endoscope washer machine (M) with an internal hot water storage tank (C);
- providing said storage tank (C) with heating means of the water contained in the storage tank;
- providing in said endoscope washer machine (M) a first hydraulic line (10) of water to be heated, coming from an external water supply network (cold water);
- providing in said first hydraulic line (10) of water to be heated:
- a water intake (A) hydraulically connected with respect to said external water supply network,
- a first solenoid valve (B) to shut off water flow, hydraulically branched downstream of said water intake (A) and maintained in a permanent hydraulically open position;
- a second solenoid valve arranged downstream of said first solenoid valve (B) and hydraulically connected to a water inlet of said storage tank (C) to load water into said storage tank (C), wherein said second solenoid valve is configured to be selectively switched into a hydraulically open position, in which it allows the water to be heated to flow from said water intake (A) to said storage tank (C), and into a hydraulically closed position, in which it shuts off the flow of water to be heated from said water intake (A) to said storage tank (C);
- switching said second solenoid valve into said open position, and filling the storage tank (C) with water from said external water supply network,
- after said storage tank (C) has been filled, switching said second solenoid valve into said closed position, in which further water cannot enter said storage tank (C) through said first hydraulic line (10);
- heating the water contained in said storage tank (C) by means of said heating means up to a temperature of 60°C or higher, maintaining this temperature for at least the time required to eliminate all possible proliferation of bacteria inside said storage tank (C);
- switching said second solenoid valve into said open position, in which water from said external water supply network is conveyed inside said storage tank (C) from said water intake (A) through said first hydraulic line (10);
- mixing water from said external water supply network entering in the storage tank (C) through said first hydraulic line (10) with the hot water present in the storage tank (C), to take the water mixed in said storage tank (C) to a temperature ranging from 30°C to 40°C;
- providing a hot water outlet (C1) of said storage tank (C) and hydraulically connecting said hot water outlet (C1) of said storage tank (C) with said tank (D) by means of a second hydraulic line (20), which passes, in a sealed manner, through said discharge opening (E) of said tank (D) and flows into said tank (D);
- providing said water outlet (C1) with flow shut-off valve means, configured to be switched selectively into a hydraulically open position, in which they allow the hot water to flow from said storage tank (C) through said second hydraulic line (20), and into a hydraulically closed position, in which they shut off the flow of hot water from said storage tank (C) through said second hydraulic line (20);
- switching said valve means of said water outlet (C1) of said storage tank (C) into a hydraulically open position and closing said discharge opening (E) of said tank (D);
- pushing the hot water contained in the storage tank (C) maintained at a temperature ranging from 30°C to 40°C through said water outlet (C1) and said second hydraulic line (20) to said tank (D), passing through the discharge opening (E) of said tank (D), by means of a difference in pressure of the cold water entering said storage tank (C) through said first hydraulic line (10);
- filling the tank (D) to the desired level with hot water having a temperature ranging from 30°C to 40°C;
- after reaching the desired level of hot water in the tank (D), switching said second solenoid valve into the hydraulically closed position and switching said valve means of said water outlet (C1) of said storage tank (C) into the hydraulically closed position;
- utilizing the hot water contained in the tank (D) to cleanse an endoscope to be reprocessed arranged in said tank (D);
- after cleansing of said endoscope to be reprocessed, opening said discharge opening (E) of said tank (D) and discharging the water from said tank (D).

2. Method according to claim 1, **characterized by** the step of providing a hydraulic branch of said first hydraulic line (10), upstream of said second solenoid valve to said tank (D), and providing said hydraulic branch with filter means, with a flow sensor and with a third solenoid valve, so that, when said second solenoid valve is switched into the hydraulically closed position, said hydraulic branch provides a passage of network water (cold water) from said network intake (A) directly into the wash tank (D), to carry out at least one of the following operations: prewash, intermediate rinse, disinfection and final rinse of an endoscope to be reprocessed in said tank (D).

3. Method according to claim 1 and/or 2, **characterized by** the step of configuring said storage tank (C) as boiler.

4. System for controlling the temperature of the water used to cleanse an endoscope to be reprocessed in an endoscope washer machine (M), comprising a tank (D) made of medical grade polymer material, wherein:
- said tank (D) is provided with an opening (E) in the bottom of the tank for discharging the water from said tank, and
- said discharge opening (E) is configured to be selectively opened and allow the outflow of water and closed to prevent the outflow of water from said tank (D);
- a hot water storage tank (C) is arranged inside said endoscope washer machine (M);
- heating means of the water contained in said storage tank (C) are arranged in said storage tank (C);
- a first hydraulic line (10) of water to be heated is arranged in said endoscope washer machine (M), is branched from an external water supply network (cold water) and comprises:
- a water intake (A) hydraulically connected with respect to said external water supply network,
- a first solenoid valve (B) to shut off water flow, hydraulically branched downstream of said water intake (A) and maintained in a permanent hydraulically open position;
- a second solenoid valve arranged downstream of said first solenoid valve (B) and hydraulically connected to a water inlet of said storage tank (C) to load water into said storage tank (C), wherein said second solenoid valve is configured to be selectively switched into a hydraulically open position, in which it allows the water to be heated to flow from said water intake (A) to said storage tank (C), and into a hydraulically closed position, in which it shuts off the flow of water to be heated from said water intake (A) to said storage tank (C);
- said heating means are configured to heat the water contained in said storage tank (C) to a temperature of 60°C or higher and maintain this temperature at least for the time required to eliminate all possible proliferation of bacteria inside said storage tank (C);
- a hot water outlet (C1) is arranged in said storage tank (C) and is hydraulically connected with said tank (D) by means of a second hydraulic line (20), which passes, in a sealed manner, through said discharge opening (E) of said tank (D) and flows out into said tank (D);
- water flow shut-off valve means are arranged in said water outlet (C1) and are configured to be electively switched into a hydraulically open position, in which they allow hot water to flow through said second hydraulic line (20), and into a hydraulically closed position, in which they shut off the flow of hot water through said second hydraulic line (20);
- wherein, in a first operating arrangement:
- said storage tank (C) is filled with water from the external water supply network, by means of said first hydraulic line (10) and said second solenoid valve switched into the hydraulically open position,
- said second solenoid valve is switched into the hydraulically closed position and the water contained in said storage tank (C) is heated to a temperature of the water of 60°C or higher,
- the water is maintained at a temperature of 60°C or higher for the time required to eliminate all possible proliferation of bacteria inside said storage tank (C),
- **characterized in that** in a second and subsequent operating arrangement:
- said second solenoid valve is switched into said hydraulically open position allowing the water of said external water supply network to flow inside said storage tank (C) from said water intake (A) through said first hydraulic line (10), mixing with the hot water present in the storage tank (C), wherein the temperature of the water mixed ranges from 30°C to 40°C;
- wherein, in a third and subsequent operating arrangement:
- said valve means of said water outlet (C1) of said storage tank (C) are arranged in a hydraulically open position and said discharge opening (E) of said tank (D) is closed, so that the hot water mixed in the storage tank (C) and maintained at a temperature ranging from 30°C to 40°C is pushed through said water outlet (C1) and said second hydraulic line (20) into said tank (D), passing through the discharge opening (E) of the tank (D), by means of a difference in pressure of the cold water entering said storage tank (C) through said first hydraulic line (10), and fills the tank (D) with hot water at a temperature ranging from 30°C to 40°C to the desired level;
- after reaching the desired level of hot water at the temperature ranging from 30°C to 40°C in said tank (D), said second solenoid valve is switched into the hydraulically closed position and said water outlet (C1) of said storage tank (C) is switched into the hydraulically closed position;
- the hot water contained in the tank (D) at a temperature ranging from 30°C to 40°C allows correct cleansing of an endoscope to be reprocessed arranged in the tank (D);
- after cleansing of the endoscope to be reprocessed and opening of said discharge opening (E) of said tank (D), the water utilized for cleansing in said tank (D) is discharged through said opening (E).

5. System according to claim 4, **characterized in that** a hydraulic branch of said first hydraulic line (10) is arranged upstream of said second solenoid valve and extends into said tank (D), wherein said hydraulic branch is provided with filter means, with a flow sensor and with a third solenoid valve, so that, when said second solenoid valve is switched into the hydraulically closed position, said hydraulic branch provides a passage of network water (cold water) from said network intake (A) directly into said washing tank (D), to carry out at least the following operations: prewash, intermediate rinse, disinfection and final rinse of a endoscope to be reprocessed in said tank (D).

6. System according to claim 4, **characterized in that** said storage tank (C) is configured as a boiler.

## Patentansprüche

1. Verfahren zur Steuerung der Temperatur des zur Reinigung eines in einer Endoskop-Waschmaschine (M) aufzubereitenden Endoskops verwendeten Wassers, wobei die Endoskop-Waschmaschine (M) einen aus medizinischem Polymermaterial bestehenden Behälter (D) umfasst, wobei:
- der Behälter (D) mit einer Öffnung (E) im Boden des Behälters zum Ablassen des Wassers aus dem Behälter versehen ist, und
- die Ablauföffnung (E) dazu ausgebildet ist, selektiv geöffnet zu werden und den Abfluss von Wasser zu ermöglichen sowie geschlossen zu werden, um den Abfluss von Wasser aus dem Behälter (D) zu verhindern; und das Verfahren die folgenden Schritte umfasst:
- Bereitstellen der Endoskop-Waschmaschine (M) mit einem internen Warmwasserspeichertank (C);
- Bereitstellen des Speichertanks (C) mit Mitteln zum Erhitzen des im Speichertank enthaltenen Wassers;
- Bereitstellen in der Endoskop-Waschmaschine (M) einer ersten hydraulischen Leitung (10) für zu erhitzendes Wasser, kommend von einem externen Wasserversorgungsnetz (Kaltwasser);
- Bereitstellen in der ersten hydraulischen Leitung (10) für zu erhitzendes Wasser:
- eines Wasserzulaufs (A), hydraulisch verbunden mit dem externen Wasserversorgungsnetz,
- eines ersten Magnetventils (B) zum Absperren des Wasserflusses, hydraulisch verzweigt stromabwärts des Wasserzulaufs (A) und in dauerhaft hydraulisch geöffneter Stellung gehalten;
- eines zweiten Magnetventils, stromabwärts des ersten Magnetventils (B) angeordnet und hydraulisch mit einem Wassereinlass des Speichertanks (C) verbunden, um Wasser in den Speichertank (C) einzuladen, wobei das zweite Magnetventil dazu ausgebildet ist, selektiv in eine hydraulisch offene Stellung geschaltet zu werden, in der es das zu erhitzende Wasser vom Wasserzulauf (A) zum Speichertank (C) strömen lässt, und in eine hydraulisch geschlossene Stellung, in der es den Fluss des zu erhitzenden Wassers vom Wasserzulauf (A) zum Speichertank (C) unterbricht;
- Umschalten des zweiten Magnetventils in die offene Stellung und Befüllen des Speichertanks (C) mit Wasser aus dem externen Wasserversorgungsnetz;
- nach Befüllen des Speichertanks (C) Umschalten des zweiten Magnetventils in die geschlossene Stellung, in der weiteres Wasser nicht über die erste hydraulische Leitung (10) in den Speichertank (C) eintreten kann;
- Erhitzen des im Speichertank (C) enthaltenen Wassers mittels der Heizmittel auf eine Temperatur von 60 °C oder höher und Aufrechterhalten dieser Temperatur mindestens für die zur Eliminierung jeglicher möglicher Bakterienvermehrung innerhalb des Speichertanks (C) erforderliche Zeit;
- Umschalten des zweiten Magnetventils in die offene Stellung, in der Wasser aus dem externen Wasserversorgungsnetz über den Wasserzulauf (A) durch die erste hydraulische Leitung (10) in den Speichertank (C) gefördert wird;
- Vermischen des aus dem externen Wasserversorgungsnetz in den Speichertank (C) durch die erste hydraulische Leitung (10) eintretenden Wassers mit dem im Speichertank (C) vorhandenen heißen Wasser, um das im Speichertank (C) gemischte Wasser auf eine Temperatur im Bereich von 30 °C bis 40 °C zu bringen;
- Bereitstellen eines Warmwasserauslasses (C1) des Speichertanks (C) und hydraulisches Verbinden des Warmwasserauslasses (C1) des Speichertanks (C) mit dem Behälter (D) mittels einer zweiten hydraulischen Leitung (20), welche in abgedichteter Weise durch die Ablauföffnung (E) des Behälters (D) hindurchgeführt ist und in den Behälter (D) mündet;
- Bereitstellen des Wasserauslasses (C1) mit Durchfluss-Absperrventilmitteln, die dazu ausgebildet sind, selektiv in eine hydraulisch offene Stellung geschaltet zu werden, in der sie den Fluss des heißen Wassers aus dem Speichertank (C) durch die zweite hydraulische Leitung (20) zulassen, und in eine hydraulisch geschlossene Stellung, in der sie den Fluss des heißen Wassers aus dem Speichertank (C) durch die zweite hydraulische Leitung (20) unterbrechen;
- Umschalten der Ventilmittel des Wasserauslasses (C1) des Speichertanks (C) in eine hydraulisch offene Stellung und Schließen der Ablauföffnung (E) des Behälters (D);
- Fördern des im Speichertank (C) enthaltenen und auf einer Temperatur im Bereich von 30 °C bis 40 °C gehaltenen heißen Wassers durch den Wasserauslass (C1) und die zweite hydraulische Leitung (20) in den Behälter (D), durch die Ablauföffnung (E) des Behälters (D) hindurch, mittels eines Druckunterschieds des durch die erste hydraulische Leitung (10) in den Speichertank (C) eintretenden Kaltwassers;
- Befüllen des Behälters (D) bis zum gewünschten Niveau mit heißem Wasser mit einer Temperatur im Bereich von 30 °C bis 40 °C;
- nach Erreichen des gewünschten Heißwasserniveaus im Behälter (D) Umschalten des zweiten Magnetventils in die hydraulisch geschlossene Stellung und Umschalten der Ventilmittel des Wasserauslasses (C1) des Speichertanks (C) in die hydraulisch geschlossene Stellung;
- Verwenden des im Behälter (D) enthaltenen heißen Wassers zur Reinigung eines in dem Behälter (D) angeordneten aufzubereitenden Endoskops;
- nach der Reinigung des aufzubereitenden Endoskops Öffnen der Ablauföffnung (E) des Behälters (D) und Ablassen des Wassers aus dem Behälter (D).

2. Verfahren nach Anspruch 1, **gekennzeichnet durch** den Schritt des Bereitstellens eines hydraulischen Abzweigs der ersten hydraulischen Leitung (10) stromaufwärts des zweiten Magnetventils zum Behälter (D), und Bereitstellen des hydraulischen Abzweigs mit Filtermitteln, mit einem Durchflusssensor und mit einem dritten Magnetventil, so dass, wenn das zweite Magnetventil in die hydraulisch geschlossene Stellung geschaltet ist, der hydraulische Abzweig einen Durchgang von Netzwerkwasser (Kaltwasser) vom Netzeinlass (A) direkt in den Waschbehälter (D) bereitstellt, um mindestens einen der folgenden Vorgänge durchzuführen: Vorwäsche, Zwischenspülung, Desinfektion und Endspülung eines in dem Behälter (D) aufzubereitenden Endoskops.

3. Verfahren nach Anspruch 1 und/oder 2, **gekennzeichnet durch** den Schritt der Ausbildung des Speichertanks (C) als Boiler.

4. System zur Steuerung der Temperatur des zur Reinigung eines in einer Endoskop-Waschmaschine (M) aufzubereitenden Endoskops verwendeten Wassers, umfassend einen aus medizinischem Polymermaterial bestehenden Behälter (D), wobei:
- der Behälter (D) mit einer Öffnung (E) im Boden des Behälters zum Ablassen des Wassers aus dem Behälter versehen ist, und
- die Ablauföffnung (E) dazu ausgebildet ist, selektiv geöffnet zu werden und den Abfluss von Wasser zu ermöglichen sowie geschlossen zu werden, um den Abfluss von Wasser aus dem Behälter (D) zu verhindern;
- ein Warmwasserspeichertank (C) innerhalb der Endoskop-Waschmaschine (M) angeordnet ist;
- Mittel zum Erhitzen des im Speichertank (C) enthaltenen Wassers im Speichertank (C) angeordnet sind;
- eine erste hydraulische Leitung (10) für zu erhitzendes Wasser in der Endoskop-Waschmaschine (M) angeordnet ist, von einem externen Wasserversorgungsnetz (Kaltwasser) abgezweigt ist und umfasst:
- einen Wasserzulauf (A), hydraulisch verbunden mit dem externen Wasserversorgungsnetz,
- ein erstes Magnetventil (B) zum Absperren des Wasserflusses, hydraulisch verzweigt stromabwärts des Wasserzulaufs (A) und in dauerhaft hydraulisch geöffneter Stellung gehalten;
- ein zweites Magnetventil stromabwärts des ersten Magnetventils (B) angeordnet und hydraulisch mit einem Wassereinlass des Speichertanks (C) verbunden, um Wasser in den Speichertank (C) einzuladen, wobei das zweite Magnetventil dazu ausgebildet ist, selektiv in eine hydraulisch offene Stellung geschaltet zu werden, in der es das zu erhitzende Wasser vom Wasserzulauf (A) zum Speichertank (C) strömen lässt, und in eine hydraulisch geschlossene Stellung, in der es den Fluss des zu erhitzenden Wassers vom Wasserzulauf (A) zum Speichertank (C) unterbricht;
- die Heizmittel dazu ausgebildet sind, das im Speichertank (C) enthaltene Wasser auf eine Temperatur von 60 °C oder höher zu erhitzen und diese Temperatur mindestens für die zur Eliminierung jeglicher möglicher Bakterienvermehrung innerhalb des Speichertanks (C) erforderliche Zeit aufrechtzuerhalten;
- ein Warmwasserauslass (C1) im Speichertank (C) angeordnet ist und hydraulisch mit dem Behälter (D) mittels einer zweiten hydraulischen Leitung (20) verbunden ist, welche in abgedichteter Weise durch die Ablauföffnung (E) des Behälters (D) hindurchgeführt ist und in den Behälter (D) mündet;
- Durchfluss-Absperrventilmittel im Wasserauslass (C1) angeordnet sind und dazu ausgebildet sind, selektiv in eine hydraulisch offene Stellung geschaltet zu werden, in der sie heißen Wasserfluss durch die zweite hydraulische Leitung (20) zulassen, und in eine hydraulisch geschlossene Stellung, in der sie den Fluss des heißen Wassers durch die zweite hydraulische Leitung (20) unterbrechen;
- wobei in einer ersten Betriebsanordnung:
- der Speichertank (C) mittels der ersten hydraulischen Leitung (10) und des in hydraulisch offene Stellung geschalteten zweiten Magnetventils mit Wasser aus dem externen Wasserversorgungsnetz gefüllt wird,
- das zweite Magnetventil in die hydraulisch geschlossene Stellung geschaltet wird und das im Speichertank (C) enthaltene Wasser auf eine Temperatur von 60 °C oder höher erhitzt wird,
- das Wasser für die zur Eliminierung jeglicher möglicher Bakterienvermehrung innerhalb des Speichertanks (C) erforderliche Zeit bei einer Temperatur von 60 °C oder höher gehalten wird, **dadurch gekennzeichnet, dass** in einer zweiten und nachfolgenden Betriebsanordnung:
- das zweite Magnetventil in die hydraulisch offene Stellung geschaltet wird, wodurch Wasser aus dem externen Wasserversorgungsnetz vom Wasserzulauf (A) durch die erste hydraulische Leitung (10) in den Speichertank (C) strömt und sich mit dem im Speichertank (C) vorhandenen heißen Wasser vermischt, wobei die Temperatur des gemischten Wassers im Bereich von 30 °C bis 40 °C liegt;
- wobei in einer dritten und nachfolgenden Betriebsanordnung:
- die Ventilmittel des Wasserauslasses (C1) des Speichertanks (C) in eine hydraulisch offene Stellung gebracht werden und die Ablauföffnung (E) des Behälters (D) geschlossen wird, so dass das im Speichertank (C) gemischte und auf einer Temperatur im Bereich von 30 °C bis 40 °C gehaltene heiße Wasser durch den Wasserauslass (C1) und die zweite hydraulische Leitung (20) in den Behälter (D) gedrückt wird, durch die Ablauföffnung (E) des Behälters (D) hindurch, mittels eines Druckunterschieds des durch die erste hydraulische Leitung (10) in den Speichertank (C) eintretenden Kaltwassers, und den Behälter (D) bis zum gewünschten Niveau mit heißem Wasser bei einer Temperatur im Bereich von 30 °C bis 40 °C füllt;
- nach Erreichen des gewünschten Niveaus von heißem Wasser mit einer Temperatur im Bereich von 30 °C bis 40 °C im Behälter (D) das zweite Magnetventil in die hydraulisch geschlossene Stellung geschaltet wird und der Wasserauslass (C1) des Speichertanks (C) in die hydraulisch geschlossene Stellung geschaltet wird;
- das im Behälter (D) enthaltene heiße Wasser bei einer Temperatur im Bereich von 30 °C bis 40 °C eine korrekte Reinigung eines im Behälter (D) angeordneten aufzubereitenden Endoskops ermöglicht;
- nach der Reinigung des aufzubereitenden Endoskops und Öffnen der Ablauföffnung (E) des Behälters (D) das zur Reinigung im Behälter (D) verwendete Wasser durch die Öffnung (E) abgelassen wird.

5. System nach Anspruch 4, **gekennzeichnet dadurch, dass** ein hydraulischer Abzweig der ersten hydraulischen Leitung (10) stromaufwärts des zweiten Magnetventils angeordnet ist und sich in den Behälter (D) erstreckt, wobei der hydraulische Abzweig mit Filtermitteln, mit einem Durchflusssensor und mit einem dritten Magnetventil versehen ist, so dass, wenn das zweite Magnetventil in die hydraulisch geschlossene Stellung geschaltet ist, der hydraulische Abzweig einen Durchgang von Netzwerkwasser (Kaltwasser) vom Netzeinlass (A) direkt in den Waschbehälter (D) bereitstellt, um mindestens die folgenden Vorgänge durchzuführen: Vorwäsche, Zwischenspülung, Desinfektion und Endspülung eines in dem Behälter (D) aufzubereitenden Endoskops.

6. System nach Anspruch 4, **gekennzeichnet dadurch, dass** der Speichertank (C) als Boiler ausgebildet ist.

## Revendications

1. Procédé de commande de la température de l'eau utilisée pour le nettoyage d'un endoscope à retraiter dans une machine de lavage d'endoscopes (M) comprenant une cuve (D) réalisée en matériau polymère de qualité médicale, dans lequel :
- ladite cuve (D) est pourvue d'une ouverture (E) dans le fond de la cuve pour l'évacuation de l'eau hors de ladite cuve, et
- ladite ouverture d'évacuation (E) est configurée pour être ouverte sélectivement et permettre l'écoulement de l'eau et fermée pour empêcher l'écoulement de l'eau hors de ladite cuve (D), et le procédé comprend les étapes consistant à :
- équiper ladite machine de lavage d'endoscopes (M) d'un réservoir interne de stockage d'eau chaude (C) ;
- équiper ledit réservoir de stockage (C) de moyens de chauffage de l'eau contenue dans le réservoir de stockage ;
- prévoir dans ladite machine de lavage d'endoscopes (M) une première conduite hydraulique (10) d'eau à chauffer, provenant d'un réseau externe d'alimentation en eau (eau froide) ;
- prévoir dans ladite première conduite hydraulique (10) d'eau à chauffer :
- une entrée d'eau (A) hydrauliquement connectée audit réseau externe d'alimentation en eau,
- une première électrovanne (B) destinée à interrompre l'écoulement de l'eau, hydrauliquement dérivée en aval de ladite entrée d'eau (A) et maintenue en position hydrauliquement ouverte permanente ;
- une deuxième électrovanne disposée en aval de ladite première électrovanne (B) et hydrauliquement connectée à une entrée d'eau dudit réservoir de stockage (C) pour charger de l'eau dans ledit réservoir de stockage (C), dans laquelle ladite deuxième électrovanne est configurée pour être commutée sélectivement dans une position hydrauliquement ouverte, dans laquelle elle permet à l'eau à chauffer de s'écouler depuis ladite entrée d'eau (A) vers ledit réservoir de stockage (C), et dans une position hydrauliquement fermée, dans laquelle elle interrompt l'écoulement de l'eau à chauffer depuis ladite entrée d'eau (A) vers ledit réservoir de stockage (C) ;
- commuter ladite deuxième électrovanne dans ladite position ouverte, et remplir le réservoir de stockage (C) avec de l'eau provenant dudit réseau externe d'alimentation en eau,
- après que ledit réservoir de stockage (C) a été rempli, commuter ladite deuxième électrovanne dans ladite position fermée, dans laquelle de l'eau supplémentaire ne peut pas entrer dans ledit réservoir de stockage (C) par ladite première conduite hydraulique (10) ;
- chauffer l'eau contenue dans ledit réservoir de stockage (C) au moyen desdits moyens de chauffage jusqu'à une température de 60 °C ou plus, en maintenant cette température pendant au moins le temps requis pour éliminer toute prolifération possible de bactéries à l'intérieur dudit réservoir de stockage (C) ;
- commuter ladite deuxième électrovanne dans ladite position ouverte, dans laquelle l'eau provenant dudit réseau externe d'alimentation en eau est amenée à l'intérieur dudit réservoir de stockage (C) depuis ladite entrée d'eau (A) par ladite première conduite hydraulique (10) ;
- mélanger l'eau provenant dudit réseau externe d'alimentation en eau entrant dans le réservoir de stockage (C) par ladite première conduite hydraulique (10) avec l'eau chaude présente dans le réservoir de stockage (C), pour amener l'eau mélangée dans ledit réservoir de stockage (C) à une température comprise entre 30 °C et 40 °C ;
- prévoir une sortie d'eau chaude (C1) dudit réservoir de stockage (C) et connecter hydrauliquement ladite sortie d'eau chaude (C1) dudit réservoir de stockage (C) avec ladite cuve (D) au moyen d'une deuxième conduite hydraulique (20), laquelle traverse, de manière étanche, ladite ouverture d'évacuation (E) de ladite cuve (D) et débouche dans ladite cuve (D) ;
- équiper ladite sortie d'eau (C1) de moyens de vanne d'arrêt de débit, configurés pour être commutés sélectivement dans une position hydrauliquement ouverte, dans laquelle ils permettent à l'eau chaude de s'écouler depuis ledit réservoir de stockage (C) à travers ladite deuxième conduite hydraulique (20), et dans une position hydrauliquement fermée, dans laquelle ils interrompent l'écoulement de l'eau chaude depuis ledit réservoir de stockage (C) à travers ladite deuxième conduite hydraulique (20) ;
- commuter lesdits moyens de vanne de ladite sortie d'eau (C1) dudit réservoir de stockage (C) dans une position hydrauliquement ouverte et fermer ladite ouverture d'évacuation (E) de ladite cuve (D) ;
- pousser l'eau chaude contenue dans le réservoir de stockage (C) maintenue à une température comprise entre 30 °C et 40 °C à travers ladite sortie d'eau (C1) et ladite deuxième conduite hydraulique (20) vers ladite cuve (D), en passant par ladite ouverture d'évacuation (E) de ladite cuve (D), au moyen d'une différence de pression de l'eau froide entrant dans ledit réservoir de stockage (C) par ladite première conduite hydraulique (10) ;
- remplir la cuve (D) jusqu'au niveau souhaité avec de l'eau chaude ayant une température comprise entre 30 °C et 40 °C ;
- après avoir atteint le niveau souhaité d'eau chaude dans la cuve (D), commuter ladite deuxième électrovanne dans la position hydrauliquement fermée et commuter lesdits moyens de vanne de ladite sortie d'eau (C1) dudit réservoir de stockage (C) dans la position hydrauliquement fermée ;
- utiliser l'eau chaude contenue dans la cuve (D) pour nettoyer un endoscope à retraiter disposé dans ladite cuve (D) ;
- après le nettoyage dudit endoscope à retraiter, ouvrir ladite ouverture d'évacuation (E) de ladite cuve (D) et évacuer l'eau hors de ladite cuve (D).

2. Procédé selon la revendication 1, **caractérisé par** l'étape consistant à prévoir une dérivation hydraulique de ladite première conduite hydraulique (10), en amont de ladite deuxième électrovanne vers ladite cuve (D), et à équiper ladite dérivation hydraulique de moyens de filtrage, d'un capteur de débit et d'une troisième électrovanne, de sorte que, lorsque ladite deuxième électrovanne est commutée dans la position hydrauliquement fermée, ladite dérivation hydraulique fournit un passage d'eau de réseau (eau froide) depuis ladite entrée de réseau (A) directement dans la cuve de lavage (D), pour effectuer au moins l'une des opérations suivantes :
prélavage, rinçage intermédiaire, désinfection et rinçage final d'un endoscope à retraiter dans ladite cuve (D).

3. Procédé selon la revendication 1 et/ou 2, **caractérisé par** l'étape consistant à configurer ledit réservoir de stockage (C) comme chaudière.

4. Système de commande de la température de l'eau utilisée pour nettoyer un endoscope à retraiter dans une machine de lavage d'endoscopes (M), comprenant une cuve (D) réalisée en matériau polymère de qualité médicale, dans lequel :
- ladite cuve (D) est pourvue d'une ouverture (E) dans le fond de la cuve pour l'évacuation de l'eau hors de ladite cuve, et
- ladite ouverture d'évacuation (E) est configurée pour être ouverte sélectivement et permettre l'écoulement de l'eau et fermée pour empêcher l'écoulement de l'eau hors de ladite cuve (D) ;
- un réservoir de stockage d'eau chaude (C) est disposé à l'intérieur de ladite machine de lavage d'endoscopes (M) ;
- des moyens de chauffage de l'eau contenue dans ledit réservoir de stockage (C) sont disposés dans ledit réservoir de stockage (C) ;
- une première conduite hydraulique (10) d'eau à chauffer est disposée dans ladite machine de lavage d'endoscopes (M), est dérivée d'un réseau externe d'alimentation en eau (eau froide) et comprend :
- une entrée d'eau (A) hydrauliquement connectée audit réseau externe d'alimentation en eau,
- une première électrovanne (B) destinée à interrompre l'écoulement de l'eau, hydrauliquement dérivée en aval de ladite entrée d'eau (A) et maintenue en position hydrauliquement ouverte permanente ;
- une deuxième électrovanne disposée en aval de ladite première électrovanne (B) et hydrauliquement connectée à une entrée d'eau dudit réservoir de stockage (C) pour charger de l'eau dans ledit réservoir de stockage (C), dans laquelle ladite deuxième électrovanne est configurée pour être commutée sélectivement dans une position hydrauliquement ouverte, dans laquelle elle permet à l'eau à chauffer de s'écouler depuis ladite entrée d'eau (A) vers ledit réservoir de stockage (C), et dans une position hydrauliquement fermée, dans laquelle elle interrompt l'écoulement de l'eau à chauffer depuis ladite entrée d'eau (A) vers ledit réservoir de stockage (C) ;
- lesdits moyens de chauffage sont configurés pour chauffer l'eau contenue dans ledit réservoir de stockage (C) jusqu'à une température de 60 °C ou plus et maintenir cette température pendant au moins le temps requis pour éliminer toute prolifération possible de bactéries à l'intérieur dudit réservoir de stockage (C) ;
- une sortie d'eau chaude (C1) est disposée dans ledit réservoir de stockage (C) et est hydrauliquement connectée avec ladite cuve (D) au moyen d'une deuxième conduite hydraulique (20), laquelle traverse, de manière étanche, ladite ouverture d'évacuation (E) de ladite cuve (D) et débouche dans ladite cuve (D) ;
- des moyens de vanne d'arrêt de débit sont disposés dans ladite sortie d'eau (C1) et sont configurés pour être commutés sélectivement dans une position hydrauliquement ouverte, dans laquelle ils permettent à l'eau chaude de s'écouler à travers ladite deuxième conduite hydraulique (20), et dans une position hydrauliquement fermée, dans laquelle ils interrompent l'écoulement de l'eau chaude à travers ladite deuxième conduite hydraulique (20) ;
- dans une première configuration de fonctionnement :
- ledit réservoir de stockage (C) est rempli avec de l'eau provenant du réseau externe d'alimentation en eau au moyen de ladite première conduite hydraulique (10) et de ladite deuxième électrovanne commutée dans la position hydrauliquement ouverte,
- ladite deuxième électrovanne est commutée dans la position hydrauliquement fermée et l'eau contenue dans ledit réservoir de stockage (C) est chauffée jusqu'à une température de 60 °C ou plus,
- l'eau est maintenue à une température de 60 °C ou plus pendant le temps requis pour éliminer toute prolifération possible de bactéries à l'intérieur dudit réservoir de stockage (C),
**caractérisé en ce que** dans une deuxième configuration de fonctionnement ultérieure :
- ladite deuxième électrovanne est commutée dans ladite position hydrauliquement ouverte permettant à l'eau dudit réseau externe d'alimentation en eau de s'écouler à l'intérieur dudit réservoir de stockage (C) depuis ladite entrée d'eau (A) par ladite première conduite hydraulique (10), en se mélangeant avec l'eau chaude présente dans le réservoir de stockage (C), dans laquelle la température de l'eau mélangée est comprise entre 30 °C et 40 °C ;
- et dans une troisième configuration de fonctionnement ultérieure :
- lesdits moyens de vanne de ladite sortie d'eau (C1) dudit réservoir de stockage (C) sont disposés dans une position hydrauliquement ouverte et ladite ouverture d'évacuation (E) de ladite cuve (D) est fermée, de sorte que l'eau chaude mélangée dans le réservoir de stockage (C) et maintenue à une température comprise entre 30 °C et 40 °C est poussée à travers ladite sortie d'eau (C1) et ladite deuxième conduite hydraulique (20) dans ladite cuve (D), en passant par ladite ouverture d'évacuation (E) de ladite cuve (D), au moyen d'une différence de pression de l'eau froide entrant dans ledit réservoir de stockage (C) par ladite première conduite hydraulique (10), et remplit ladite cuve (D) avec de l'eau chaude à une température comprise entre 30 °C et 40 °C jusqu'au niveau souhaité ;
- après avoir atteint le niveau souhaité d'eau chaude à une température comprise entre 30 °C et 40 °C dans ladite cuve (D), ladite deuxième électrovanne est commutée dans la position hydrauliquement fermée et ladite sortie d'eau (C1) dudit réservoir de stockage (C) est commutée dans la position hydrauliquement fermée ;
- l'eau chaude contenue dans ladite cuve (D) à une température comprise entre 30 °C et 40 °C permet un nettoyage correct d'un endoscope à retraiter disposé dans ladite cuve (D);
- après nettoyage de l'endoscope à retraiter et ouverture de ladite ouverture d'évacuation (E) de ladite cuve (D), l'eau utilisée pour le nettoyage dans ladite cuve (D) est évacuée à travers ladite ouverture (E).

5. Système selon la revendication 4, **caractérisé en ce qu'**une dérivation hydraulique de ladite première conduite hydraulique (10) est disposée en amont de ladite deuxième électrovanne et s'étend dans ladite cuve (D), dans lequel ladite dérivation hydraulique est pourvue de moyens de filtrage, d'un capteur de débit et d'une troisième électrovanne, de sorte que, lorsque ladite deuxième électrovanne est commutée dans la position hydrauliquement fermée, ladite dérivation hydraulique fournit un passage d'eau de réseau (eau froide) depuis ladite entrée de réseau (A) directement dans ladite cuve de lavage (D), pour effectuer au moins les opérations suivantes : prélavage, rinçage intermédiaire, désinfection et rinçage final d'un endoscope à retraiter dans ladite cuve (D).

6. Système selon la revendication 4, **caractérisé en ce que** ledit réservoir de stockage (C) est configuré comme chaudière.
